Europäisches Patentamt

(19) European Patent Office

Office européen des brevets

(11) **EP 0 408 770 B1**

(12) **EUROPEAN PATENT SPECIFICATION**

(45) Date of publication and mention
of the grant of the patent:
**05.06.1996 Bulletin 1996/23**

(21) Application number: **90902694.0**

(22) Date of filing: **06.02.1990**

(51) Int. Cl.⁶: $C08B\ 37/00$, $A61K\ 31/725$

(86) International application number:
**PCT/JP90/00141**

(87) International publication number:
**WO 90/08784 (09.08.1990 Gazette 1990/19)**

(54) **NEW SULFATED POLYSACCHARIDE, PHARMACEUTICALLY ACCEPTABLE SALTS THEREOF, PREPARATION THEREOF, AND DRUG CONTAINING THE SAME AS ACTIVE INGREDIENT**

SULFATIERTES POLYSACCHARID, DESSEN PHARMAZEUTISCH VERTRÄGLICHE SALZE, DESSEN HERSTELLUNG UND MEDIKAMENT, DAS DIESES ALS WIRKSSTOFF ENTHÄLT

NOUVEAU POLYSACCHARIDE SULFATE, SES SELS PHARMACEUTIQUEMENT ACCEPTABLES, SA PREPARATION, ET MEDICAMENT LE CONTENANT A TITRE D'INGREDIENT ACTIF

(84) Designated Contracting States:
**AT BE CH DE DK ES FR GB IT LI LU NL SE**

(30) Priority: **06.02.1989 JP 28299/89**

(43) Date of publication of application:
**23.01.1991 Bulletin 1991/04**

(73) Proprietor: **TAIHO PHARMACEUTICAL COMPANY LIMITED**
**Tokyo 101 (JP)**

(72) Inventors:
• **FAN, Hui-Zeng**
**508, Gate 3**
**Yao Yan Li**
**Tianjin (CN)**
• **YU, Song**
**706, Building 4**
**Cadre Sanatorium**
**Tianjin (CN)**
• **YAMANAKA, Etsuji**
**Saitama 367 (JP)**
• **NUMATA, Kazuhiro**
**Saitama 367 (JP)**
• **OKA, Toshinori**
**14-17, Aza Seicho**
**Shozui**
**Aizumicho**
**Tokushima 771-12 (JP)**
• **SUZUKI, Norihiko**
**501, Kagasuno**
**Kawauchicho**
**Tokushima 771-01 (JP)**

• **MURANAKA, Yoshiyuki**
**682-5, Enokize**
**kawauchicho**
**Tokushima 771-01 (JP)**

(74) Representative: **VOSSIUS & PARTNER**
**Postfach 86 07 67**
**81634 München (DE)**

(56) References cited:
GB-A- 2 098 232          JP-A- 6 122 021
JP-A-63 128 001

• **THE JOURNAL OF BIOLOGICAL CHEMISTRY, vol. 263, no. 34, 5th December 1988, pages 18176-18183, The American Society for Biochemistry and Molecular Biology, Inc., US; R.P. VIEIRA et al.: "Occurrence of a unique fucose-branched chondroitin sulfate in the body wall of a sea cucumber"**
• **CHEMICAL ABSTRACTS No. CA109(5): 31852c (Zhongguo Yaolixue Yu Dulixue Zazhi, 2(2), 98-101 (1988) Zhang, Peiwen; Luo, Sufang; Zhong, Chunning; Wang, Qianji: Abstract of "Anticoagulant effect of Holothuria Leucospilota Acid Mucopolysaccharide".**
• **CHEMICAL ABSTRACTS No. CA107(21): 190638n (Zhongguo Yaoli Xuebao, 8(5), 447-50 (1987) Shan, Chunwen; Liang, Qizhao; Wang, Qiangji:Abstract of "Effects of an Acidic Polysaccharide from Holothuria Leucospilota on the Platelet Aggregation in Rabbits.")**

EP 0 408 770 B1

- CHEMICAL ABSTRACTS No. CA99(3): 19901k (Yaoxue Xuebao, 18(3), 203-8 (1983) Fan, Huizeng; Chen, Judi; Lu, Peihong; Hao, Xiaoge; Li, Haitang: Abstract of "Acidic Polysaccharides from Holothuria Leucospilota (Brandt).")
- CHEMICAL ABSTRACTS No. CA97(3): 20682e (Yaoxue Tongbao, 16(10), 631 (1981) Fan, Huizeng; Chen, Judi; Lu, Peihong; Li, Haitang: Abstract of "Acidic Polysaccharides from Holothuria Leucospilota.")
- CHEMICAL ABSTRACTS No. CA94(8): 52763m(Yao Hsueh Hsueh Pao, 15(5), 263-70 (1980) Fan, Hui - Zeng; Chen, Ju - Di; Lin, KE - Zhong: Abstract of "Isolation of an Acidic Mucopolysaccharide from Stichopus Japonicus Selenka and Some of its Physical and Chemical Properties.")
- CHEMICAL ABSTRACTS No. CA84(25): 175525m (Chem. Pharm. Bull., 24(2), 275-84 (1976) KITAGAWA, I.; SUGAWARA, T.; YOSIOKA, I.: Abstract of "Saponin and Sapogenol. XV. Antifungal Glycosides from the Sea Cucumber Stichopus Japonicus Selenka".)

Remarks:
The file contains technical information submitted after the application was filed and not included in this specification

## Description

The present invention relates to a novel sulfated polysaccharide, a pharmaceutically acceptable salt thereof, a process for preparing the same and a medicament containing the same as an effective component.

The present inventors separated a sulfated polysaccharide from the body wall of a sea cucumber by extraction with alkali, the sulfated polysaccharide having an anti-coagulation activity and a lipid clearing activity which are typical of heparin. The inventors named the polysaccharide FGAG (Yao Hsueh 1980, 15(5), 263-270, Zhongyao Tongbao 1982, 7(4), 27-29, Hsueh Pao 1983, 18(3), 203-208 and Japanese Unexamined Patent Publication No.63-128001). Japanese Unexamined Patent Publication No.63-10601 discloses another example of the separation of sulfated polysaccharide by other researchers. Although differently named, the sulfated polysaccharides described in the above prior art publications are all identical and have the following physicochemical constants.

Characteristic:

white, amorphous, highly hygroscopic powder

Molecular weight:

about 15,000 to about 80,000 (as measured by gel filtration)

Analysis for composition: As shown below.

| Galactosamine | 13 to 17 wt.% |
|---|---|
| Glucuronic acid | 16 to 19 wt.% |
| Fucose | 13 to 27 wt.% |
| Sulfate | 27 to 38.5 wt.% |

Molar ratio: As shown below.

Galactosamine: glucuronic acid: fucose: sulfate = 1 : 1±0.2 : 1.35±0.35 : 3.6±0.6

According to the above analytical values and the like, FGAG is identified as a high-molecular weight sulfated polysaccharide comprising galactosamine, glucuronic acid, fucose, etc. and is characterized by a larger content of sulfate than known natural sulfated polysaccharides.

With a high anti-coagulation activity, said FGAG was once a candidate for a medicament for curing disseminated intravascular coagulation (DIC). However, FGAG was later found to have a high activity to cause platelet aggregation when used for human beings and to be useless in treatment of humans' DIC if used as it is because of such side effect.

The Journal of Biological Chemistry, Vol. 263, No. 34 (1988), 18176-18183, describes the occurrence of different fractions of sulfated polysaccharides in the body wall of the sea cucumber. The fractions vary in their glucuronic acid, galactosamine, fucose and sulfate content.

JP-A-63128001 discloses polysaccharides, e.g. FGAG with a molecular weight of 30,000 to 80,000 Daltons, with high platelet aggragation activity.

Chemical Abstract, Vol. 99, 1983, page 394, 19901K discloses two fucose-containing acidic polysaccharides, HL-S and HL-P, which are obtained from the dried body wall of H. leucospilota. HL-P inhibits thrombin in vitro and also has an inhibitory effect on tumor growth in mice.

GB-A-2098232 discloses the preparation of injectable chondroitin sulfate by depolymerization using hydrogen peroxide.

In view of the above situation, the present inventors conducted extensive research on compounds which can be used as an excellent medicament for treatment of DIC and which have activities like heparin's. Our finding was that the sulfated polysaccharide prepared by depolymerization of FGAG or a salt thereof exhibits substantially no activity to cause platelet aggregation while sustaining the anti-coagulation activity and other activities like heparin's. We further discovered that unlike heparin, the sulfated polysaccharide shows an activity to inhibit the production of thrombin without displaying an anti-Xa or anti-thrombin activity, and thus may be potentially effective in treatment of thrombosis. The present inventors named the novel sulfated polysaccharide D-HG.

The present invention has been accomplished based on these novel findings.

According to the invention, there are provided a novel sulfated polysaccharide (D-HG), and a pharmaceutically acceptable salt thereof, a process for preparing the same and a medicament for treatment of DIC and thrombosis containing the above as the effective component.

D-HG of the invention has the physicochemical properties shown below.

[1] Molecular weight: 3,000 to 24,300 daltons (as measured by high performance GPC)
[2] Characteristic: white, amorphous, highly hygroscopic powder
[3] Solubility: soluble in water but insoluble in ethanol and acetone

[4] Specific rotation: $[\alpha]_D^{20}$ = -55 to -73° (C = 1%)

[5] Color reaction: As shown below

| | |
|---|---|
| Elson-Morgan reaction | + |
| Carbazole-sulfuric acid reaction | + |
| Cysteine-sulfuric acid reaction | + |
| Orcinol-hydrochloric acid reaction | + |
| Azure A metachromasia reaction | + |

[6] Analysis for composition: D-HG comprises constituent saccharides including galactosamine (abbreviated to GalN), glucuronic acid (abbreviated to GA) and fucose (abbreviated to Fuc) and sulfate in a molar ratio of GalN : GA : Fuc : sulfate = 1 : 0.8 ± 0.2 : 0.85 ± 0.15 : 3.4 ± 0.9.

Analyses were conducted by the following methods to check galactosamine, glucuronic acid, fucose and sulfate.

GalN:

White method (Carbohydrate Research, 114: 586, 201)

GA:

Bitter-Muir method (Anal. Biochem., 4: 330, 1962)

Fuc:

Dische method (J. Biol. Chem., 175: 595, 1948)

Sulfate:

Dodgson & Price method (Biochem. J., 84: 106, 1962)

The above analytic results show that D-HG has in the molecule sulfate and carboxyl group which react with bases to form a salt. D-HG is stable in the form of a salt and isolated and purified usually in the form of a salt. Usable as salts are pharmaceutically acceptable salts including salts of potassium, sodium or like alkali metals, and salts of calcium, magnesium, barium or like alkaline earth metals, or pyridinium salt or like organic bases. Shown below is the composition of constituent saccharides in a form in which a salt is not formed, i.e., in free form.

| | |
|---|---|
| GalN | 18 to 24 wt.% |
| GA | 14 to 21 wt.% |
| Fuc | 13 to 20 wt.% |
| Sulfate | 31 to 44 wt.% |

A preferred molecular weight of D-HG and a salt thereof is about 4,000 to about 15,000 (as determined by high performance GPC).

D-HG of the invention is prepared from FGAG as a starting material. For preparation of D-HG, FGAG or a salt thereof is depolymerized, followed by isolation and purification. FGAG is obtained by decomposing the body wall of a sea cucumber, an oceanic life, with alkali, and further decomposing the resulting product with pancreatin or like proteolytic enzymes for extraction, followed by isolation and purification.

FGAG or a salt thereof for use in the preparation of D-HG of the invention can be easily produced by the methods disclosed in the known publications cited above in reference to the prior art, more specifically for example by the method to be described later in Reference Example. Examples of sea cucumbers useful in the preparation of FGAG or a salt thereof are:

Stichopus japonicus Selenka,

Stichopus chloronoyus Brandt,

Stichopus variegatus Semper,

Holothuria pervicax Selenka,

Holothuria atra,

Holothuria argus,

Holothuria edulis,

Holothuria scabra,

Parastichopus nigripunctatus,

Thelenota ananas,

Holothuria monacaria Lesson,

Holothuria leucospilota Brandt,

Cucumaria chronhjelmi,

Cucumaria echinata,
Cucumaria frondosa Japonica,
Pentacta australis,
Paracaudina chilensis ransonneti,
Molpadia musculus,
Leptosynapta inhaerens,
Polycheira rufescens,
Synapta maculata,
Halodeima cinerascens (Brandt),
Actinopyga lacanora (Jaeger),
Actinopyga echinites (Jaeger),
Microthele nobilis (Selenka), etc.

The sea cucumber to be used as the starting material may be a raw or dried one. Of the sea cucumbers exemplified above, Stichopus japonicus Selenka is most preferred as the starting material.

D-HG is prepared by dissolving the above-obtained FGAG or a salt thereof in water and depolymerizing the solution. In the depolymerization reaction, a high-molecular weight sulfated polysaccharide such as heparin or the like is converted into a low-molecular weight sulfated polysaccharide. A depolymerizing agent is usually used in the reaction. Examples of useful depolymerizing agents are hydrogen peroxide, hypochlorous acid, hypobromus acid, sodium hypochlorate and like hypohalogenous acids and salts thereof; periodic acid, sodium periodate and like periodic acids and salts thereof, etc. Ascorbic acid, ferrous ion or the like is usable as a reaction accelerator. Optionally, the depolymerization reaction may be effected by application of radiations such as ultrasonic waves, ultraviolet rays, gamma rays or the like alone in lieu of a depolymerizing agent or in combination with the above depolymerizing agent. The most preferred depolymerization method in the invention is one using hydrogen peroxide as a depolymerizing agent. Hydrogen peroxide is reacted in an amount of 1 to 31 wt.%, preferably 1 to 16 wt.% in terms of a hydrogen peroxide concentration. The reaction time is usually 1 to 60 hours, preferably 3 to 40 hours, and the reaction temperature ranges from room temperature to about 80°C, preferably about 40 to about 60°C. The pH range in the reaction of hydrogen peroxide is acidic or neutral in the range of from 1 to 8, preferably 3 to 7. To maintain a constant pH value, hydrogen peroxide may be reacted in a buffer such as acetate buffer, phosphate buffer, Tris buffer or the like, or a pH controller using diluted sodium hydroxide or the like may be used in the reaction. On completion of the reaction, the pH is returned to neutral range, and isolation and purification are conducted. The isolation and purification can be done, for example, by fractional precipitation using an organic solvent such as ethanol or acetone; acetate such as potassium acetate, barium acetate, calcium acetate or ammonium acetate; or quaternary ammonium salt such as cetyltrimethyl ammonium salt.

The isolation and purification is also feasible by ion exchange chromatography using resins such as DEAE-Cellulose (product of Sigma Chemical Co.), DEAE-Toyopearl (product of Tosoh Corporation), DEAE-Cellulofine (product of Chisso Corporation) or Dowex-I (product of Dow Chemical Co.), or gel filtration chromatography using resins such as Sephadex G-50, Sephadex G-200 (both products of Pharmacia-LKB Biotechnology), by dialysis using Spectra/Por (product of Spectrum Medical Industries, Inc.) or by ultrafiltration. These means are employed alone or in a suitable combination thereof. Ion exchange chromatography, gel filtration chromatography and ultrafiltration are preferable to produce easily D-HG having no activity to cause platelet aggregation.

The thus obtained D-HG is usually isolated in the form of a salt of sodium and/or potassium.

D-HG in a salt form can be transformed into free D-HG by treatment with a cation-exchange resin such as Dowex 50W. D-HG in a salt form, if necessary, can be converted into a desired pharmaceutically acceptable salt by salt exchange commonly employed. Usable as salts of sulfated polysaccharides are pharmaceutically acceptable salts including salts of potassium, sodium or like alkali metals, and salts of calcium, magnesium, barium or like alkaline earth metals, or pyridinium salt or like organic bases.

The treatment of DIC and thrombosis by D-HG of the invention is conducted utilizing its anti-coagulation activity against the acceleration of coagulation in blood vessels which causes DIC and thrombosis. The range of anti-coagulation activity of D-HG includes an activity to inhibit the platelet aggregation by thrombin as well as an anti-coagulation enzyme activity, typically an activity to prolong the activated partial thromboplastin time. The anti-coagulation activity of D-HG is entirely different from heparin's in that D-HG does not require any plasma factor such as anti-thrombin III in exhibiting the activity nor is influenced by the anti-heparin factor such as platelet factor 4. A further difference from heparin is that D-HG has an activity to inhibit the production of thrombin without displaying an anti-Xa activity or an anti-thrombin activity, hence apparently effective against thrombosis. The feature of D-HG is that unlike heparin and FGAG, D-HG has substantially no activity to cause platelet coagulation, which is the fatal activity of medicaments for treatment of DIC and thrombosis. The expression "substantially no activity to cause platelet coagulation" means that when administered to organisms, especially human beings, D-HG does not exhibit platelet coagulation which poisons organisms or aggravates thrombosis.

D-HG is made into various pharmaceutical compositions useful for DIC and thrombosis treatment. Stated more specifically, the composition comprising an effective amount of D-HG and/or a pharmaceutically acceptable salt and a pharmaceutically acceptable carrier can be prepared in various administration forms. The administration form can be any of tablets, capsules, powders, granules, grains, solutions, emulsions, suspensions and like oral forms, and injections, suppositories, ointment, plaster and like parenteral forms. These preparations can be manufactured by conventional methods already known to those skilled in the art. A solid preparation for oral administration can be prepared by mixing the effective component of the invention with an excipient with or without addition of binders, disintegrators, lubricants, coloring agents, flavorings, perfumes, etc. and making the mixture into tablets, capsules, powders, granules, grains or the like in a conventional manner. Injection preparations can be produced by adding a pH-adjusting agent, buffer, stabilizer, isotonizing agent, local anesthetic and the like to the effective component, and making the mixture into intravenous, intramuscular, subcutaneous, intracutaneous or intraperitoneal injections in a conventional manner. Suppositories can be prepared by making a mixture of the effective component, base materials and optionally a surfactant and the like into a suppository in a conventional manner.

Examples of excipients useful for oral solid preparations are lactose, sucrose, starch, talc, magnesium stearate, crystalline cellulose, methyl cellulose, carboxymethyl cellulose, glycerin, sodium alginate, gum arabic. Examples of binders useful for oral preparations include polyvinyl alcohol, polyvinyl ether, ethyl cellulose, gum arabic, shellac, sucrose. Examples of useful lubricants are magnesium stearate or talc. The coloring agents, disintegrators and other auxiliaries to be added include those commonly used in the art. Tablets may be coated by well-known methods.

Examples of base materials useful for suppositories include oily base materials such as macrogol, lanolin, cacao oil, fatty acid triglyceride, Witepsol (registered trademark for the product of Dynamite Nobel).

The amount of the effective component per each unit dosage varies with the symptoms of the patient to be given the preparation, the form of the preparation, etc. Usually a preferred amount is 10 to 200 mg in an oral preparation, 1 to 100 mg in an injection, or 10 to 100 mg in a suppository, per unit dosage. The daily clinical dosage of the composition of the invention also varies with the patient's age, sex, conditions and other factors but usually may be in the range of about 10 to about 1,000 mg, preferably about 50 to about 200 mg in terms of the effective component and can be given at 1 to 4 divided doses.

According to the present invention, there is provided a novel sulfated polysaccharide, D-HG, having substantially no activity to cause platelet coagulation and having an excellent anti-coagulation activity and remarkable characteristics as a medicament for treatment of DIC and thrombosis.

The present invention will be described in greater detail with reference to Reference Example, Examples and Pharmacological Tests. The percentages in Reference Example and Examples are all by weight.

Reference Example 1

Preparation of FGAG

One kilogram of dried Stichopus japonicus was immersed in 10 ℓ of warm water and left to swell overnight. The flesh was removed and homogenized. Potassium hydroxide was added in an amount to give a 1N mixture. The mixture was treated at 60°C for 100 minutes and adjusted to a pH of 8.5. After addition of 50 g of pancreatin, the mixture was stirred at 50°C for 3 hours.

After removal of impurities by centrifugation, 4.3 ℓ of ethanol was added to the residue. The mixture was allowed to stand at 4°C and the resulting precipitate was collected. The precipitate was washed with 80% ethanol, anhydrous ethanol and acetone in this sequence, and dried under reduced pressure, giving 50 g of a crude product. Fifty grams of the crude product was dissolved in 3.5 ℓ of water and the solution was centrifuged to remove the insolubles. To the supernatant were added 5% sodium chloride and 40% ethanol to give a precipitate. The precipitate was collected by centrifugation. After the precipitate was dissolved in 2.5 ℓ of water, the solution was adjusted to a pH of 10.5. To the solution was added dropwise a 30% aqueous solution of hydrogen peroxide. The mixture was decolorized in a water bath at 50°C with heating for about 3 hours. After cooling, the insolubles were removed by centrifugation. To the supernatant was added about 490 g of potassium acetate and the mixture was kept at 4°C overnight. The following day, the resulting precipitate was dissolved in 2 ℓ of water, the solution was cooled to 0°C, and the pH was adjusted to 2.8. The insolubles were removed from the solution by centrifugation. After neutralizing the supernatant, 196 g of potassium acetate was added. The mixture was allowed to stand at 4°C to give a precipitate, which was then collected by centrifugation. The precipitate was dissolved in water to give a solution having a potassium acetate concentration of 0.5M and the solution was left overnight at 4°C. The precipitate was collected by centrifugation, washed with 40% methanol and dissolved in 1 ℓ of water. To the solution were added 5% sodium chloride and 40% ethanol to give a precipitate. The precipitate was collected by centrifugation, washed with 80% methanol, anhydrous ethanol and acetone in this sequence, and dried under reduced pressure, giving 17 g of a FGAG sodium/potassium salt. The physicochemical constants of the salt are as follows.

Molecular weight:

55,000 (as determined by high performance GPC)

Analysis for composition: As shown below

| GalN: | 20.0 % |
|---|---|
| GA: | 18.6 % |
| Fuc: | 17.2 % |
| Sulfate: | 36.6 % |
| Na: | 6.2 % |
| K: | 7.4 % |

Example 1

Ten grams of the FGAG sodium/potassium salt obtained in Reference Example 1 was dissolved in 75 ml of water and 25 ml of a 30% aqueous solution of hydrogen peroxide was added. While maintaining the solution at a pH of about 7 with a diluted sodium hydroxide solution using a pH controler, the solution was heated at 60°C for 12 hours. After cooling, 2% sodium chloride and 40% ethanol were added to give a precipitate. The precipitate was collected by centrifugation, washed with 80% ethanol, anhydrous ethanol and acetone in this sequence, and dried under reduced pressure, giving 7.15 g of a D-HG sodium/potassium salt.

Example 2

A 6.95 g quantity of a D-HG sodium/potassium salt was prepared by the same procedure as in Example 1 with the exception of treatment with hydrogen peroxide for 24 hours.

Example 3

A D-HG sodium/potassium salt was prepared by the same procedure as in Example 1 with the exception of conducting the reaction while maintaining the pH of about 4 with a diluted alkali solution. Yield 6.4 g.

Example 4

Ten grams of the FGAG sodium/potassium salt obtained in Reference Example 1 was dissolved in 83.3 ml of a 0.2M phosphate buffer (pH 7.0). To the solution was added 16.7 ml of a 30% aqueous solution of hydrogen peroxide. The mixture was treated at 60°C for 12 hours. After cooling, 2% sodium chloride and 40% ethanol were added to give a precipitate. The precipitate was collected by centrifugation, washed with 80% ethanol, anhydrous ethanol and acetone in this sequence, and dried under reduced pressure, giving 7.18 g of a D-HG sodium/potassium salt.

Example 5

A D-HG sodium/potassium salt was prepared by the same procedure as in Example 4 with the exception of conducting the reaction using a 0.2M acetate buffer (pH 3.5). Yield 7.05 g.

Examples 6 and 7

Two grams of the FGAG sodium/potassium salt obtained in Reference Example 1 was dissolved in 15 ml of water. To the solution was added 5 ml of a 30% aqueous solution of hydrogen peroxide, and the mixture was treated at 60°C for 14 hours (Example 6) or 40 hours (Example 7). After cooling, the mixture was adjusted to a pH of 7 to 8 and thoroughly dialyzed against water using Spectra/por 3. The mixture was lyophilized and dried under reduced pressure. In this way, 1.62 g and 1.76 g of D-HG sodium/potassium salts were prepared.

Example 8

Two grams of the FGAG sodium/potassium salt obtained in Reference Example 1 was dissolved in 16.7 ml of water. To the solution was added 3.3 ml of a 30% aqueous solution of hydrogen peroxide, and the mixture was treated at 45°C for 24 hours. After cooling, the mixture was returned to a pH of about 7 after which 2% sodium chloride and 40% ethanol were added to provide a precipitate. The precipitate was collected by centrifugation, washed with 80% ethanol, anhydrous ethanol and acetone in this sequence, and dried under reduced pressure, giving 1.41 g of a D-HG sodium/potassium salt.

Examples 9 to 12

Two grams of the FGAG sodium/potassium salt obtained in Reference Example 1 was dissolved in 15 ml of water. To the solution was added 5 ml of a 30% aqueous solution of hydrogen peroxide, and the mixture was treated at 60°C for 4, 8, 12 or 24 hours. After cooling, the mixture was adjusted to a pH of 7 to 8 and fully dialyzed against water using Spectra/por 3. The same treatment as in Example 8 followed. In this way, 1.42 g, 1.35 g, 1.35 g and 1.2 g of D-HG sodium/potassium salts were produced.

Examples 13 and 14

Two grams of the FGAG sodium/potassium salt obtained in Reference Example 1 was dissolved in 14.7 ml of water. To the solution was added 5.3 ml of a 30% aqueous solution of hydrogen peroxide. The mixture was treated at 45°C for 14 hours or 40 hours. After cooling, the mixture was returned to a pH of about 7 after which 2% sodium chloride and 40% ethanol were added to give a precipitate. The same treatment as in Examples 6 to 7 followed. In this way, 1.64 g and 1.62 g of D-HG sodium/potassium salts were prepared.

Example 15

Two grams of the FGAG sodium/potassium salt obtained in Reference Example 1 was dissolved in 30 ml of water and treated for 12 hours in the same manner as in Example 8. The solution was fractionated with a solution of sodium chloride on a Sephadex G-50T column (product of Pharmacia-LKB Biotechnology). While monitoring uronic acid, peaks were divided into three. The eluate obtained last was collected, fully dialyzed against water, lyophilized and dried under reduced pressure, giving 0.2 g of a D-HG sodium salt.

Example 16

A 0.5 g quantity of the D-HG sodium/potassium salt obtained in Example 8 was fractionated in the same manner as in Example 15, giving 0.18 g of a D-HG sodium salt.

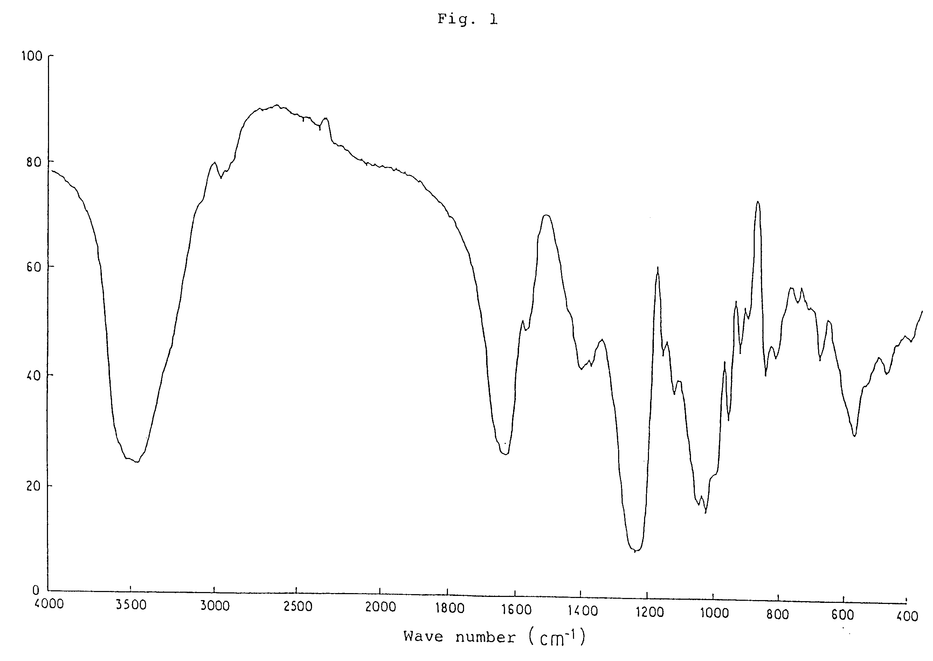

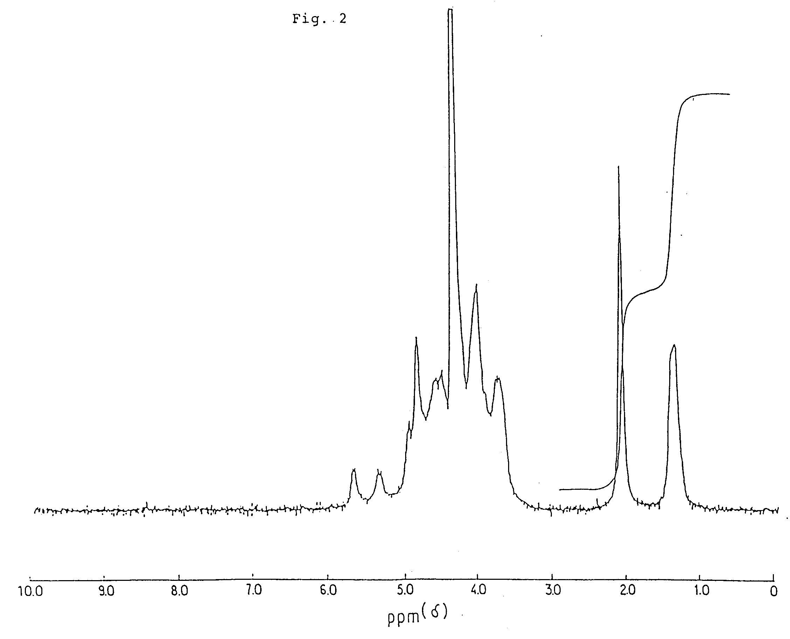

Fig. 1 shows an infrared absorption spectrum of the D-HG sodium salt (as measured with KBr tablet) and Fig. 2 a proton nuclear magnetic resonance spectrum (in $D_2O$, 90MHz, 70°C) thereof.

Example 17

Two grams of the FGAG sodium/potassium salt obtained in Reference Example 1 was dissolved in 16.7 ml of water, and 3.3 ml of a 30% aqueous solution of hydrogen peroxide was added. The mixture was treated at 45°C for 9 hours. After cooling, the mixture was returned to a pH of about 7 after which 2% sodium chloride and 40% ethanol were added to give a precipitate. The precipitate was collected by centrifugation, washed with 80% ethanol, anhydrous ethanol and acetone in this sequence, and dried under reduced pressure giving 1.54g of a D-HG sodium/potassium salt.

Example 18

Two grams of the FGAG sodium/potassium salt obtained in Reference Example 1 was treated for 12 hours by the same method as in Example 17, giving 1.52 g of a D-HG sodium/potassium salt.

Example 19

One gram of the FGAG sodium/potassium salt obtained in Reference Example 1 was dissolved in 8.7 ml of 0.2M phosphate buffer (pH 7.0). To the solution was added 1.3 ml of a 30% aqueous solution of hydrogen peroxide and the mixture was treated at 60°C for 3 hours. After cooling, 5% sodium chloride and 66% ethanol were added to the mixture to give a precipitate. The precipitate was collected by centrifugation, washed with 80% ethanol, anhydrous ethanol and diethyl ether in this sequence, and dried under reduced pressure, giving a D-HG sodium/potassium salt. The obtained salt was dissolved in 10 ml of 20mM Tris-HCl buffer (pH 7.0) and the solution was admixed with DEAE-Toyopearl (product of Tosoh Corporation) thoroughly equilibrated with the buffer. Elution was conducted in the buffer with a linear concentration gradient of sodium chloride (0 to 1 M). While monitoring uronic acid, peak fractions were collected and precipitation occurred with addition of a two-fold amount of ethanol. The precipitate was collected by centrifugation, washed with 80% ethanol, anhydrous ethanol and diethyl ether in this order and dried under reduced pressure, giving a D-HG sodium salt. Yield 0.39 g.

Table 1 shows physicochemical properties of D-HG's obtained in the above Examples.

Table 1

| Ex. | MW × 10³ | $[\alpha]_D^{20}$ | Composition | | | | | | Molar ratio | | | |
|---|---|---|---|---|---|---|---|---|---|---|---|---|
| | | | GalN | Fuc | GA | Sul* | Na | K | GalN : | GA : | Fuc : | Sul* |
| 1 | 10.2 | −71.2 | 21.7 | 16.7 | 16.5 | 29.7 | 7.1 | 6.4 | 1 : | 0.70 : | 0.84 : | 2.5 |
| 2 | 9.7 | −67.1 | 19.8 | 15.8 | 15.1 | 30.2 | 7.2 | 7.9 | 1 : | 0.70 : | 0.87 : | 2.8 |
| 3 | 14.1 | −71.5 | 20.2 | 17.6 | 15.6 | 33.1 | 6.2 | 6.7 | 1 : | 0.71 : | 0.95 : | 3.0 |
| 4 | 4.7 | −55.4 | 19.8 | 15.1 | 13.3 | 31.4 | 8.2 | 4.8 | 1 : | 0.62 : | 0.83 : | 2.9 |
| 5 | 6.5 | −61.2 | 20.6 | 15.6 | 15.8 | 32.8 | 7.8 | 4.4 | 1 : | 0.71 : | 0.83 : | 2.9 |
| 6 | 12.0 | −70.8 | 18.6 | 16.5 | 17.1 | 38.6 | 4.7 | 6.1 | 1 : | 0.85 : | 0.97 : | 3.8 |
| 7 | 7.8 | −68.0 | 17.5 | 13.1 | 16.1 | 37.1 | 5.4 | 6.4 | 1 : | 0.85 : | 0.82 : | 3.9 |
| 8 | 13.4 | −70.1 | 17.1 | 14.6 | 14.6 | 32.7 | 8.1 | 5.5 | 1 : | 0.79 : | 0.93 : | 3.5 |
| 9 | 14.1 | −69.6 | 19.9 | 14.3 | 14.8 | 34.0 | 7.6 | 5.2 | 1 : | 0.69 : | 0.79 : | 3.1 |
| 10 | 8.6 | −66.3 | 20.8 | 15.1 | 14.3 | 34.8 | 8.5 | 5.5 | 1 : | 0.64 : | 0.79 : | 3.1 |

## Table 1 (Continued)

| Ex. | MW $\times 10^3$ | $[\alpha]_D^{20}$ | GalN | Fuc | GA | Sul* | Na | K | GalN : GA : Fuc : Sul* |
|---|---|---|---|---|---|---|---|---|---|
| 11 | 7.5 | −66.0 | 17.8 | 13.2 | 13.2 | 32.9 | 6.4 | 5.1 | 1 : 0.68 : 0.81 : 3.4 |
| 12 | 5.6 | −64.8 | 20.1 | 14.7 | 13.0 | 33.4 | 4.1 | 5.1 | 1 : 0.60 : 0.80 : 3.0 |
| 13 | 10.8 | −72.2 | 18.1 | 14.8 | 16.7 | 37.1 | 5.0 | 6.5 | 1 : 0.85 : 0.89 : 3.8 |
| 14 | 6.6 | −68.4 | 17.3 | 12.8 | 15.9 | 35.3 | 5.6 | 6.7 | 1 : 0.85 : 0.81 : 3.7 |
| 15 | 10.2 | −68.2 | 19.1 | 15.8 | 14.4 | 32.9 | 8.6 | 0 | 1 : 0.69 : 0.90 : 3.1 |
| 16 | 7.7 | −67.5 | 17.6 | 13.9 | 14.1 | 32.1 | 4.4 | 0 | 1 : 0.74 : 0.86 : 3.3 |
| 18 | 24.3 | −72.6 | 18.0 | 15.9 | 16.6 | 33.4 | 6.2 | 5.2 | 1 : 0.85 : 0.96 : 3.4 |
| 19 | 20.1 | −71.1 | 18.3 | 14.4 | 14.8 | 33.0 | 7.5 | 5.0 | 1 : 0.75 : 0.86 : 3.3 |
| 20 | 12.8 | −62.4 | 19.3 | 14.5 | 16.2 | 34.3 | 10.7 | 0 | 1 : 0.78 : 0.81 : 3.3 |

Note: In Table 1, the molecular weight (MW) was determined by high performance GPC. Sul* stands for sulfate.

D-HG's obtained in Examples 1 to 19 showed single spots in electrophoresis (Dietrich. C. P. J. Chromatogr., 130, 299 (1977)).

Preparation Example 1

Injection preparation

D-HG sodium salt prepared in Example 16 was dissolved in distilled water for injection to give a 5% aqueous solution. A 50 mg quantity (in terms of D-HG) of the solution was filled into a vial to perform lyophilization. A 2 ml quantity of physiological saline was added as a solvent.

Preparation Example 2

Injection preparation

An injection preparation was prepared according to the formulation as shown below.

| | |
|---|---|
| D-HG sodium/potassium salt (Example 12) | 40 mg |
| Physiological saline | q.s. |
| Per ampule | 2 ml |

Preparation Example 3

Tablet

Tablets were prepared according to the formulation as shown below.

| | |
|---|---|
| D-HG sodium/potassium salt (Example 14) | 10 mg |
| Corn starch | 65 mg |
| Carboxymethyl cellulose | 20 mg |
| Polyvinyl pyrrolidone | 3 mg |
| Magnesium stearate | 2 mg |
| Per tablet | 100 mg |

Preparation Example 4

Suppository

A suppository was prepared according to the formulation as shown below.

| | |
|---|---|
| D-HG sodium/potassium salt (Example 4) | 50 mg |
| Witepsol W-35 (Product of Dynamite-Nobel AG) | 950 mg |
| Per suppository | 1000 mg |

Pharmacological Test

⟨Effect on DIC model⟩

D-HG, FGAG and heparin were tested for an effect on DIC model in accordance with the method described in Japan J. Pharmacol, 35, 203-227 (1984).

Used as D-HG was the sodium salt obtained in Example 16, as FGAG the sodium/potassium salt obtained in Reference Example 1 and as a heparin a sodium salt having a potency of 185.6 U/mg.

A 800 U/kg of thrombin was intravenously injected into ICR mice (10 to 15 mice a group). After 24 hours, the fatality of mice caused by DIC was observed to calculate the survival rate. The D-HG sodium salt, FGAG sodium/potassium salt or heparin sodium salt was intravenously injected one minute before the administration of thrombin. Table 2 shows the results.

Table 2

| Medicament | Dose (mg/kg) | Survival rate (%) |
|---|---|---|
| Control | 0 | 13 |
| D-HG sodium salt | 3 | 90 |
|  | 1 | 60 |
|  | 0.3 | 40 |
| FGAG sodium/potassium salt | 1 | 60 |
| Heparin sodium salt | 1 | 80 |

D-HG produced the same anti-DIC effect as heparin and FGAG when used in an amount of 1 mg/kg. This model also serves as a thrombosis model, hence effective against thrombosis.

⟨Anti-coagulation Activity⟩

The D-HG sodium salt (Example 16) or D-HG sodium/potassium salt (Example 11) was added to citric acid-containing plasma obtained from a rabbit to a concentration of 10 $\mu$g/ml. The activity to prolong the activated partial thromboplastin time (APTT) against the control (physiological saline) was observed. Table 3 shows the results.

Table 3

| Medicament | APTT ($\Delta$ sec) |
|---|---|
| Ex. 16 | 24.7 |
| Ex. 11 | 16.2 |
| Control | 0.0 |

D-HG exhibited a remarkable anticoagulation activity.

⟨Anti-coagulation Activity in Human⟩

Using the citric acid-containing plasma obtained from more than 6 normal persons, the D-HG sodium salt (Example 16), a FGAG sodium/potassium salt and a heparin sodium salt were each observed for the activity in respect of anticoagulation parameters ($\mu$g/ml). Table 4 shows the results.

x2APTT shows the concentration ($\mu$g/ml) required to double the activated partial thromboplastin time of the control (without addition of a medicament).

IIaIC$_{90}$ is a concentration ($\mu$g/ml) for 90% inhibition of thrombin activity which was calculated by measuring the activity to prolong the thrombin time.

XaIC$_{50}$ is a medicament concentration ($\mu$g/ml) for 50% inhibition of decomposition of synthetic substrate S2222 with a factor X.

VIII IC$_{80}$ is a medicament concentration ($\mu$g/ml) for 80% inhibition of factor VIII which was calculated by measuring the activity to prolong the contact-activated coagulation time in the presence of a small amount of factor VIII using a factor VIII deficient plasma.

IIaGI is the concentration ($\mu$g/ml) required to double the control's time for complete inactivation of prothrombin in the contact-activated plasma. This represents an activity to inhibit the thrombin generation.

Table 4

| Medicament | x2APTT | IIaIC$_{90}$ | XaIC$_{50}$ | VIII IC$_{80}$ | IIaGI |
|---|---|---|---|---|---|
| Heparin sodium salt | 1.2 | 0.3 | 3.4 | 0.79 | 1.2 |
| FGAG sodium/potassium salt | 2.4 | 2 | 5200 | 1.68 | 2.4 |
| Ex. 16 | 12.0 | 30 | 5100 | 4.77 | 12.0 |

As seen from the APTT prolonging activity, D-HG sodium salts have an anti-coagulation activity, but unlike heparin sodium salts, have substantially no anti-thrombin activity or anti-factor Xa activity. On the other hand, D-HG sodium salts show an activity to inhibit the thrombin generation, which confirms that the salts have an anti-thrombosis activity. This activity is presumably due to the inhibition of factor VIII activity and the inhibition of positive feedback mechanism of coagulation cascade. The above indicate that D-HG is a remarkably unique agent for treatment of DIC or thrombosis.

〈Inhibitory Activity against Thrombin-induced Platelets Aggregation〉

The result of addition of the D-HG sodium salt obtained in Example 16 or a heparin sodium salt having a potency of 185.6 U/mg was evaluated by observing the aggregation of platelets (expressed in an increase of light transmittance) caused by the addition of 0.1 U/ml of thrombin to a suspension of plasma-free washed platelet obtained from a rabbit. Table 5 shows the results.

Table 5

| Medicament | Concentration (μg/ml) | Light transmittance (%) |
|---|---|---|
| Control | 0 | 86.6 |
| D-HG sodium salt | 3 | 46.4 |
|  | 10 | 5.2 |
| Heparin sodium salt | 3 | 80.2 |

D-HG, unlike heparin, showed a remarkable inhibitory activity against thrombin aggregation in a plasma-free system. Thus, it was confirmed that the activity of D-HG is independent of the plasma factors such as ATIII, etc.

〈Activity to Cause Platelets Aggregation in Human〉

A citrated platelet-rich plasma was obtained from five normal persons (B, E, G, H, J). The D-HG sodium salt obtained in Example 16 or FGAG sodium/potassium salt obtained in Reference Example 1 was added to the plasma, and the resulting activity to cause platelet aggregation (expressed in an increase of light transmittance) was evaluated by observation. Table 6 shows the results.

Table 6

| Medicament | Concentration (mg/ml) | Light transmittance (%) | | | | |
|---|---|---|---|---|---|---|
|  |  | B | E | G | H | J |
| D-HG sodium salt | 1 | 1.2 | 2.5 | 3.8 | 3.0 | 1.3 |
| FGAG sodium/potassium salt | 0.3 | 20.9 | 83.8 | 48.2 | 65.4 | 77.4 |
| Control | - | 2.4 | 1.3 | 2.3 | 1.8 | 2.4 |

D-HG did not have an activity to cause platelet aggregation in a concentration of 1 mg/ml, but FGAG exhibited an activity to cause platelet aggregation in a lower concentration of 300 μg/ml.

**Claims**

**Claims for the following Contracting States : AT, BE, CH, LI, DE, DK, FR, GB, IT, LU, NL, SE**

1. A sulfated polysaccharide (D-HG) and a pharmaceutically acceptable salt thereof obtainable by depolymerization of FGAG, which is a high-molecular weight sulfated polysaccharide comprising galactosamine, glucuronic acid, fucose, or a salt thereof, said sulfated polysaccharide (D-HG) having substantially no activity to cause platelet aggregation whereby the activity is measured at a concentration of 1 mg/ml as increase of light transmittance of a platelet suspension, and having the following physicochemical properties:

   [1] Molecular weight:
   3,000 to 24,300 daltons (as measured by high performance GPC)
   [2] Characteristic:
   white, amorphous, highly hygroscopic powder
   [3] Solubility:
   soluble in water but insoluble in ethanol and acetone
   [4] Specific rotation:
   $[\alpha]_D^{20}$ = -55 to -73° (C = 1%)
   [5] Color reaction: as shown below

   | | |
   |---|---|
   | Elson-Morgan reaction | + |
   | Carbazole-sulfuric acid reaction | + |
   | Cysteine-sulfuric acid reaction | + |
   | Orcinol-hydrochloric acid reaction | + |
   | Azure A metachromasia reaction | + |

   [6] Analysis for composition: as shown below Galactosamine : Glucuronic acid : Fucose : Sulfate in a molar ratio of 1 : 0.8 ± 0.2 : 0.85 ± 0.15 : 3.4 ± 0.9.

2. The sulfated polysaccharide (D-HG) and a salt thereof according to claim 1 which has a molecular weight of 4,000 to 15,000 daltons as measured by high performance GPC.

3. A process for preparing a sulfated polysaccharide (D-HG) having the physicochemical properties according to claim 1 and a salt thereof the process comprising the steps of:

   - depolymerizing FGAG or a salt thereof by the addition of a depolymerizing agent selected from the group consisting of hydrogen peroxide, hypochlorous acid, hypobromus acid, sodium hypochlorite, periodic acid and sodiumperiodate or by the application of ultrasonic waves, ultraviolet rays, or gamma rays, alone or used in combination with the depolymerizing agent; and
   - separating and purifying the resulting product by (i) fractional precipitation in the presence of acetate or an organic solvent of (ii) gel filtration or (iii) ion exchange.

4. The process according to claim 3 wherein the separation and purification are carried out by fractional precipitation using potassium acetate, ethanol or a combination of potassium acetate and ethanol.

5. The process according to claim 3 wherein the separation and purification are carried out by a gel filtration.

6. The process according to claim 3 wherein the separation and purification are carried out by an ion exchange chromatography.

7. A medicament for treatment of disseminated intravascular caagulation comprising an effective amount of the sulfated polysaccharide (D-HG) and/or the salt thereof as defined in claim 1 and a pharmaceutically acceptable carrier.

8. A medicament for treatment of thrombosis comprising an effective amount of the sulfated polysaccharide (D-HG) and/or the salt thereof as defined in claim 1 and a pharmaceutically acceptable carrier.

9. Use of the sulfated polysaccharide (D-HG) and/or the salt thereof as defined in claim 1 for preparation of a pharmacological composition for treatment of disseminated intravascular coagulation syndrome.

10. Use of the sulfated polysaccharide (D-HG) and/or the salt thereof as defined in claim 1 for preparation of a pharmacological composition for treatment of thrombosis.

11. The sulfated polysaccharide (D-HG) and salt thereof according to claim 1 wherein said FGAG or a salt thereof is a sulfated polysaccharide having a molecular weight of about 15,000 to about 80,000 daltons as measured by gel filtration.

12. The sulfated polysaccharide and salt thereof according to claim 11 wherein said FGAG has a composition comprising galactosamine 13 to 17 wt.%, glucuronic acid 16 to 19 wt.%, fucose 13 to 27 wt.%, sulfate 27 to 38.5 wt.%.

13. The sulfated polysaccharide and salt thereof according to claim 12 wherein said FGAG or a salt thereof has a molar ratio of galactosamine: glucuronic acid: fucose:sulfate of about 1 : 1 ± 0.2 : 1.35 ± 0.35 : 3.6 ± 0.6.

14. The process according to claim 3 wherein said FGAG or a salt thereof is a sulfated polysaccharide having a molecular weight of about 15,000 to about 80,000 daltons as measured by gel filtration.

15. The process according to claim 14 wherein said FGAG has a composition comprising galactosamine 13 to 17 wt.%, glucuronic acid 16 to 19 wt.%, fucose 13 to 27 wt.%, sulfate 27 to 38.5 wt.%.

16. The process according to claim 15 wherein said FGAG or a salt thereof has a molar ratio of galactosamine: glucuronic acid: fucose:sulfate of about 1 : 1 ± 0.2 : 1.35 ± 0.35 : 3.6 ± 0.6.

**Claims for the following Contracting State : ES**

1. A process for preparing a sulfated polysaccharide (D-HG) said sulfated polysaccharide (D-HG) having substantially no activity to cause platelet aggregation whereby the activity is measured at a concentration of 1 mg/ml as increase of light transmittance of a platelet suspension, and having the following physicochemical properties:

[1] Molecular weight:
3,000 to 24,300 daltons (as measured by high performance GPC)
[2] Characteristic:
white, amorphous, highly hygroscopic powder
[3] Solubility:
soluble in water but insoluble in ethanol and acetone
[4] Specific rotation:
$[a]_D^{20}$ = -55 to -73° (C = 1%)
[5] Color reaction: as shown below

| | |
|---|---|
| Elson-Morgan reaction | + |
| Carbazole-sulfuric acid reaction | + |
| Cysteine-sulfuric acid reaction | + |
| Orcinol-hydrochloric acid reaction | + |
| Azure A metachromasia reaction | + |

[6] Analysis for composition: as shown below Galactosamine : Glucuronic acid : Fucose : Sulfate in a molar ratio of 1 : 0.8 ± 0.2 : 0.85 ± 0.15 : 3.4 ± 0.9 and a pharmaceutically acceptable salt thereof, comprising the steps of depolymerizating FGAG

which is a high-molecular weight sulfated polysaccharide comprising galactosamine, glucuronic acid, fucose, or a salt thereof, and subjecting the resulting product to separation and purification.

2. A process according to claim 1 wherein the sulfated polysaccharide (D-HG) has the molecular weight of 4,000 to 15,000 (as measured by high performance GPC).

3. A process according to claim 1 or 2 wherein the separation and purification are carried out by fractional precipitation using potassium acetate or precipitation using ethanol.

4. A process according to claim 1 or 2 wherein the separation and purification are carried out by a gel filtration.

EP 0 408 770 B1

5. A process according to claim 1 or 2 wherein the separation and purification are carried out by an ion exchange chromatography.


**Patentansprüche**


**Patentansprüche für folgende Vertragsstaaten : AT, BE, CH, LI, DE, DK, FR, GB, IT, LU, NL, SE**


1. Sulfatiertes Polysaccharid (D-HG) und ein pharmazeutisch verträgliches Salz davon, erhältlich durch Depolymerisation von FGAG, das ein hochmolekulares sulfatiertes Polysaccharid ist, umfassend Galactosamin, Glucoronsäure, Fucose, oder eines Salzes davon, wobei das sulfatierte Polysaccharid (D-HG) im wesentlichen keine Aktivität in der Verursachung von Thrombocytenaggregation aufweist, wobei die Aktivität bei einer Konzentration von 1 mg/ml als Anstieg der Lichttransmission einer Thrombocytensuspension gemessen wird, und mit den folgenden physikalisch-chemischen Eigenschaften:

   [1] Molekulargewicht:
   3000 bis 24 300 Dalton (gemessen durch Hochleistungs-GPC)
   [2] Merkmale:
   weißes, amorphes, stark hygroskopisches Pulver
   [3] Löslichkeit:
   löslich in Wasser, aber unlöslich in Ethanol und Aceton
   [4] Spezifische Drehung:
   $[\alpha]_D^{20}$ = -55 bis -73° (c = 1 %)
   [5] Farbreaktion: wie nachstehend gezeigt

   | | |
   |---|---|
   | Elson-Morgan-Reaktion | + |
   | Carbazol-Schwefelsäure-Reaktion | + |
   | Cystein-Schwefelsäure-Reaktion | + |
   | Orcinol-Salzsäure-Reaktion | + |
   | Azur A-Metachromasie-Reaktion | + |

   [6] Analyse der Zusammensetzung: wie nachstehend gezeigt Galactosamin : Glucoronsäure : Fucose : Sulfat in einem Molverhältnis von 1 : 0,8 ± 0,2 : 0,85 ± 0,15 : 3,4 ± 0,9.

2. Sulfatiertes Polysaccharid (D-HG) und ein Salz davon nach Anspruch 1, das ein Molekulargewicht von 4000 bis 15 000 Dalton aufweist, gemessen durch HochleistungsGPC.

3. Verfahren zur Herstellung eines sulfatierten Polysaccharids (D-HG) mit den physikalisch-chemischen Eigenschaften nach Anspruch 1 und eines Salzes davon, wobei das Verfahren folgende Schritte umfaßt:

   - Depolymerisation von FGAG oder einem Salz davon durch die Zugabe eines Depolymerisierungsmittels, ausgewählt aus Wasserstoffperoxid, unterchloriger Säure, unterbromiger Säure, Natriumhypochlorit, Periodsäure und Natriumperiodat oder durch die Anwendung von Ultraschallwellen, UV-Strahlen oder Gammastrahlen, allein oder in Kombination mit dem Depolymerisierungsmittel; und
   - Abtrennung und Reinigung des erhaltenen Produkts durch (i) fraktionierte Fällung in Gegenwart von Acetat oder einem organischen Lösungsmittel, (ii) Gelfiltration oder (iii) Ionenaustausch.

4. Verfahren nach Anspruch 3, wobei die Abtrennung und Reinigung durch fraktionierte Fällung unter Verwendung von Kaliumacetat, Ethanol oder einer Kombination von Kaliumacetat und Ethanol durchgeführt werden.

5. Verfahren nach Anspruch 3, wobei die Abtrennung und Reinigung durch Gelfiltration ausgeführt werden.

6. Verfahren nach Anspruch 3, wobei die Abtrennung und Reinigung durch Ionenaustauschchromatographie durchgeführt werden.

7. Medikament zur Behandlung von disseminierter intravasaler Koagulation, umfassend eine wirksame Menge des sulfatierten Polysaccharids (D-HG) und/oder des Salzes davon gemäß der Definition in Anspruch 1 und einen pharmazeutisch verträglichen Träger.

16

**8.** Medikament zur Behandlung von Thrombose, umfassend eine wirksame Menge des sulfatierten Polysaccharids (D-HG) und/oder des Salzes davon gemäß der Definition in Anspruch 1 und einen pharmazeutisch verträglichen Träger.

**9.** Verwendung des sulfatierten Polysaccharids (D-HG) und/oder des Salzes davon gemäß der Defirition in Anspruch 1 für die Herstellung eines Arzneimittels zur Behandlung des disseminierte intravasaler Koagulation-Syndroms.

**10.** Verwendung des sulfatierten Polysaccharids (D-HG) und/oder des Salzes davon gemäß der Definition in Anspruch 1 zur Herstellung eines Arzneimittels zur Behandlung von Thrombose.

**11.** Sulfatiertes Polysaccharid (D-HG) und Salz davon nach Anspruch 1, wobei das FGAG oder ein Salz davon ein sulfatiertes Polysaccharid mit einem Molekulargewicht von etwa 15 000 bis etwa 80 000 Dalton, gemessen durch Gelfiltration, ist.

**12.** Sulfatiertes Polysaccharid und Salz davon nach Anspruch 11, wobei das FGAG eine Zusammensetzung aufweist, die 13 bis 17 Gew.-% Galactosamin, 16 bis 19 Gew.-% Glucuronsäure, 13 bis 27 Gew.-% Fucose und 27 bis 38,5 Gew.-% Sulfat umfaßt.

**13.** Sulfatiertes Polysaccharid und Salz davon nach Anspruch 12, wobei das FGAG oder ein Salz davon ein Molverhältnis von Galactosamin : Glucuronsäure : Fucose : Sulfat von etwa $1 : 1 \pm 0,2 : 1,35 \pm 0,35 : 3.6 \pm 0,6$ aufweist.

**14.** Verfahren nach Anspruch 3, wobei das FGAG oder ein Salz davon ein sulfatiertes Polysaccharid mit einem Molekulargewicht von etwa 15 000 bis etwa 80 000 Dalton, gemessen durch Gelfiltration, ist.

**15.** Verfahren nach Anspruch 14, wobei das FGAG eine Zusammensetzung aufweist, die 13 bis 17 Gew.-% Galactosamin, 16 bis 19 Gew.-% Glucuronsäure, 13 bis 27 Gew.-% Fucose und 27 bis 38,5 Gew.-% Sulfat umfaßt.

**16.** Verfahren nach Anspruch 15, wobei das FGAG oder ein Salz davon ein Molverhältnis von Galactosamin : Glucuronsäure : Fucose : Sulfat von etwa $1 : 1 \pm 0,2 : 1,35 \pm 0,35 : 3,6 \pm 0,6$ aufweist.

**Patentansprüche für folgenden Vertragsstaat : ES**

**1.** Verfahren zur Herstellung eines sulfatierten Polysaccharids (D-HG), wobei das sulfatierte Polysaccharid (D-HG) im wesentlichen keine Aktivität in der Verursachung von Thrombocytenaggregation aufweist, wobei die Aktivität bei einer Konzentration von 1 mg/ml als Anstieg der Lichttransmission einer Thrombocytensuspension gemessen wird, und die folgenden physikalisch-chemischen Eigenschaften hat:

[1] Molekulargewicht:
3000 bis 24 300 Dalton (gemessen durch Hochleistungs-GPC)
[2] Merkmale:
weißes, amorphes, stark hygroskopisches Pulver
[3] Löslichkeit:
löslich in Wasser, aber unlöslich in Ethanol und Aceton
[4] Spezifische Drehung:
$[\alpha]_D^{20}$ = -55 bis -73° (c = 1 %)
[5] Farbreaktion: wie nachstehend gezeigt

| | |
|---|---|
| Elson-Morgan-Reaktion | + |
| Carbazol-Schwefelsäure-Reaktion | + |
| Cystein-Schwefelsäure-Reaktion | + |
| Orcinol-Salzsäure-Reaktion | + |
| Azur A-Metachromasie-Reaktion | + |

[6] Analyse der Zusammensetzung: wie nachstehend gezeigt Galactosamin : Glucoronsäure : Fucose : Sulfat in einem Molverhältnis von $1 : 0,8 \pm 0,2 : 0,85 \pm 0,15 : 3,4 \pm 0,9$.

und eines pharmazeutisch verträglichen Salzes davon umfassend die Schritte der Depolymerisation von FGAG, das ein hochmolekulares sulfatiertes Polysaccharid ist, umfassend Galactosamin, Glucoronsäure, Fucose, oder eines Salzes davon und der Abtrennung und Reinigung des erhaltenen Produkts.

**2.** Verfahren nach Anspruch 1, wobei das sulfatierte Polysaccharid (D-HG) ein Molekulargewicht von 4000 bis 15 000 Dalton aufweist (gemessen durch HochleistungsGPC).

**3.** Verfahren nach Anspruch 1 oder 2, wobei die Abtrennung und Reinigung durch fraktionierte Fällung unter Verwendung von Kaliumacetat oder durch Fällung unter Verwendung von Ethanol durchgeführt wird.

**4.** Verfahren nach Anspruch 1 oder 2, wobei die Abtrennung und Reinigung durch Gelfiltration ausgeführt werden.

**5.** Verfahren nach Anspruch 1 oder 2, wobei die Abtrennung und Reinigung durch Ionenaustauschchromatographie durchgeführt werden.

**Revendications**

**Revendications pour les Etats contractants suivants : AT, BE, CH, LI, DE, DK, FR, GB, IT, LU, NL, SE**

**1.** Polysaccharide sulfaté (D-HG) et sel de celui-ci pharmaceutiquement acceptable, pouvant être obtenus par dépolymérisation du FGAG qui est un polysaccharide sulfaté à haut poids moléculaire comprenant la galactosamine, l'acide glucuronique, le fucose, ou un sel de ceux-ci, ledit polysaccharide sulfaté (D-HG) n'ayant pratiquement aucune activité du point de vue agrégation plaquettaire, grâce à quoi l'activité est mesurée à une concentration de 1 mg/ml comme étant l'élévation de la transmittance lumineuse d'une suspension plaquettaire, et qui ont les propriétés physico-chimiques suivantes :

[1] Poids moléculaire :
3.000 à 24.300 daltons (mesuré par chromatographie phase vapeur haute performance)
[2] Caractéristiques :
poudre blanche, amorphe, fortement hygroscopique
[3] Solubilité :
soluble dans l'eau mais insoluble dans l'éthanol et dans l'acétone
[4] Pouvoir rotatoire spécifique
$[\alpha]_D^{20} = -55$ à $-73°$ (c = 1 %)
[5] Réaction colorée : voir ci-dessous

| Réaction d'Elson-Morgan | + |
| Réaction carbazole/acide sulfurique | + |
| Réaction cystéine/acide sulfurique | + |
| Réaction orcinol/acide chlorhydrique | + |
| Réaction au Métachromasia Azur A | + |

[6] Analyse de la composition : voir ci-dessous Galactosamine/Acide glucuronique/Fucose/Sulfate, dans un ratio molaire de $(1)/(0,8 \pm 0,2)/(0,85 \pm 0,15)/(3,4 \pm 0,9)$

**2.** Polysaccharide sulfaté (D-HG) et sel de celui-ci selon la revendication 1, qui ont un poids moléculaire compris entre 4.000 et 15.000 daltons, mesuré par chromatographie phase vapeur haute performance.

**3.** Procédé de préparation d'un polysaccharide sulfaté (D-HG) ayant les propriétés physicochimiques selon la revendication 1, et d'un sel de celui-ci, le procédé comprenant les étapes :

- de dépolymérisation du FGAG, ou d'un sel de celui-ci, par addition d'un agent dépolymérisant choisi au sein du groupe comprenant le peroxyde d'hydrogène, l'acide hypochloreux, l'acide hypobromeux, l'hypochlorite de sodium, l'acide periodique, et le periodate de sodium, ou par application d'ondes ultrasonores, de rayons ultraviolets, ou de rayons gamma, seuls, ou en association avec l'agent dépolymérisant : et
- de séparation et de purification du produit obtenu

    (i) par précipitation fractionnée en présence d'un acétate ou d'un solvant organique :
    (ii) par filtration sur gel :
    (iii) par échange d'ions.

**4.** Procédé selon la revendication 3, dans lequel la séparation, et la purification sont effectuées par précipitation fractionnée en utilisant l'acétate de potassium, l'éthanol, ou une association d'acétate de potassium et d'éthanol.

**5.** Procédé selon la revendication 3 dans lequel la séparation et la purification sont effectuées par filtration sur gel.

**6.** Procédé selon la revendication 3, dans lequel la séparation et la purification sont effectuées par une chromatographie d'échange d'ions.

**7.** Médicament pour le traitement de la coagulation intravasculaire disséminée, comprenant une quantité efficace du polysaccharide sulfaté (D-HG), et/ou du sel de celui-ci selon la revendication 1, et un véhicule pharmaceutiquement acceptable.

**8.** Médicament pour le traitement de la thrombose, comprenant une quantité efficace du polysaccharide sulfaté (D-HG), et/ou du sel de celui-ci selon la revendication 1, et un véhicule pharmaceutiquement acceptable.

**9.** Utilisation du polysaccharide sulfaté (D-HG), et/ou du sel de celui-ci selon la revendication 1, pour la préparation d'une composition pharmacologique pour le traitement du syndrome de la coagulation intravasculaire disséminee.

**10.** Utilisation du polysaccharide sulfaté (D-HG), et/ou du sel de celui-ci selon la revendication 1, pour la préparation d'une composition pharmacologique pour le traitement de la thrombose.

**11.** Polysaccharide sulfaté (D-HG) et sel de celui-ci selon la revendication 1, dans lesquels ledit FGAG ou un sel de celui-ci, est un polysaccharide sulfaté ayant un poids moléculaire compris entre environ 15.000 et environ 80.000, mesuré par filtration sur gel.

**12.** Polysaccharide sulfaté (D-HG) et sel de celui-ci selon la revendication 11, dans lesquels ledit FGAG se compose de 13 à 17 % en poids, de galactosamine, de 16 à 19 % en poids, d'acide glucuronique, de 13 à 27 % en poids, de fucose, et de 27 à 38,5 % en poids, de sulfate.

**13.** Polysaccharide sulfaté (D-HG) et sel de celui-ci selon la revendication 12, dans lesquels ledit FGAG, ou un sel de celui-ci, a un ratio molaire Galactosamine / Acide glucuronique / Fucose / Sulfate, d'environ (1)/(1 ± 0,2)/(1,35 ± 0,35)/(3,6 ± 0,6).

**14.** Procédé selon la revendication 3, dans lequel ledit FGAG, ou un sel de celui-ci, est un polysaccharide sulfaté ayant un poids moléculaire compris entre 15.000 environ, et 80.000 daltons environ, mesuré par filtration sur gel.

**15.** Procédé selon la revendication 14, dans lequel ledit FGAG se compose de 13 à 17 % en poids, de galactosamine, de 16 à 19 % en poids, d'acide glucuronique, de 13 à 27 % en poids, de fucose, et de 27 à 38,5 % en poids, de sulfate.

**16.** Procédé selon la revendication 15, dans lequel ledit FGAG, ou un sel de celui-ci, a un ratio molaire Galactosamine / Acide glucuronique / Fucose / Sulfate, d'environ (1)/(1 ± 0,2)/(1,35 ± 0.35)/(3,6 ± 0,6).

**Revendications pour l'Etat contractant suivant : ES**

**1.** Procédé pour la préparation d'un polysaccharide sulfaté (D-HG), ledit polysaccharide (D-HG) n'ayant pratiquement pas d'activité d'agrégation plaquettaire, grâce à quoi l'activité est mesurée à une concentration de 1 mg/ml en termes d'augmentation de la transmittance de lumiére d'une suspension de plaquettes, et possédant les propriétés physicochimiques suivantes :

[1] Poids moléculaire :
3.000 à 24.300 daltons (mesuré par CPV haute performance)
[2] Caracteristiques :
poudre blanche, amorphe, fortement hygroscopique
[3] Solubilité :
soluble dans l'eau, mais insoluble dans l'éthanol, et l'acétone
[4] Pouvoir rotatoire spécifique :
$[\alpha]_D^{20}$ = -55 à -73° (C = 1 %)
[5] Réactions colorées : voir ci-dessous

| | |
|---|---|
| Réaction d'Elson-Morgan | + |
| Réaction carbazole-acide sulfurique | + |
| Réaction cystéine-acide sulfurique | + |

Réaction orcinol-acide chlorhydrique     +

Réaction au Métachromasia Azur A     +

[6] Analyse de la composition : voir ci-dessous Galactosamine/Acide glucuronique/Fucose/Sulfate dans un ratio molaire de 1/0,8 (± 0,2)/0,85 (± 0,15)/3,4 (± 0,9)

et d'un sel de celui-ci, pharmaceutiquement acceptable, comprenant les étapes de dépolymérisation du FGAG qui est un polysaccharide sulfaté à haut poids moléculaire contenant de la galactosamine, de l'acide glucuronique, du fucose ou d'un sel de celui-ci, et de soumission du produit ainsi obtenu, à une séparation, et à une purification.

2.   Procédé selon la revendication 1, dans lequel le polysaccharide sulfaté (D-HG) a un poids moléculaire de 4.000 à 15.000 (mesuré par CPV haute performance).

3.   Procédé selon la revendication 1, ou la revendication 2, dans lequel la séparation et la purification sont effectués par précipitation fractionnée à l'acétate de potassium, ou par précipitation à l'éthanol.

4.   Procédé selon la revendication 1, ou la revendication 2, dans lequel la séparation et la purification sont effectuées par filtration sur gel.

5.   Procédé selon la revendication 1, ou la revendication 2, dans lequel la séparation et la purification sont effectuées par chromatographie d'échange d'ions.

Fig. 1

Fig. 2